# EUROPÄISCHE PATENTSCHRIFT

(11) **EP 2 442 726 B1**
(45) Veröffentlichungstag und Bekanntmachung des Hinweises auf die Patenterteilung: **04.05.2016**
(21) Anmeldenummer: 10725395.7
(22) Anmeldetag: 17.06.2010
(51) Int. Cl.: A61B 10/00, A61L 31/16, A61M 1/36, G01N 33/543

(54) **DETEKTIONSVORRICHTUNG ZUR IN VIVO UND/ODER IN VITRO ANREICHERUNG VON PROBENMATERIAL**
DETECTION DEVICE FOR IN VIVO AND/OR IN VITRO ENRICHMENT OF SAMPLE MATERIAL
DISPOSITIF DE DÉTECTION POUR ENRICHIR IN VIVO ET/OU IN VITRO UN MATÉRIAU D'ÉCHANTILLON

(30) Priorität: 17.06.2009 EP 09075271
(43) Veröffentlichungstag der Anmeldung: 25.04.2012
(73) Patentinhaber: GILUPI GmbH, 14476 Potsdam OT Golm (DE)
(72) Erfinder: LÜCKE, Klaus, 14476 Potsdam OT GoIm (DE); BOLLMANN, Andreas, 14476 Potsdam OT GoIm (DE); MEWES, Steffi, 14476 Potsdam OT GoIm (DE); NIESTROJ, Robert, 14476 Potsdam OT GoIm (DE)
(74) Vertreter: Grünecker Patent- und Rechtsanwälte PartG mbB
(86) Internationale Anmeldenummer: PCT/EP2010/003644
(87) Internationale Veröffentlichungsnummer: WO 2010/145824

(56) Entgegenhaltungen:
- WO-A1-2006/131400
- WO-A1-2010/025719
- WO-A2-03/065881
- US-A1- 2006 051 265
- US-A1- 2007 191 932
- Florek: "The acute systemic toxicity study for normal catheterand cell-select catheter (CSC)" Archives of Perinatal Medicine Bd. 14, Nr. 2, 2008, XP002598285 Gefunden im Internet: URL:http://www.ptmp.pl/archives/apm/14-2/A PM142-3-Florek.pdf [gefunden am 2010-08-26]
- Izabela Firkowska ET AL: "Biocompatible Nanomaterials and Nanodevices Promising for Biomedical Applications" In: "Nanomaterials for Application in Medicine and Biology", 1 January 2008 (2008-01-01), Springer Netherlands, Dordrecht, XP055119345, ISSN: 1874-6500 ISBN: 978-1-40-206829-4 pages 1-15, DOI: 10.1007/978-1-4020-6829-4_1,
- KLAUS LÜCKE: "Nanodetector for Collecting Rare Cells Out of the Circulating Blood", BIOFORUM LODZ, 3 June 2009 (2009-06-03), XP055220843,

## Beschreibung

Die Erfindung betrifft eine Detektionsvorrichtung zur in vivo und/oder in vitro Anreicherung von Probenmaterial, umfassend eine mit Detektionsrezeptoren bestückte Funktionsfläche.

Die WO 2006/131400 A1 offenbart einen Biosensor mit zweidimensionalen, bogenförmigen Nanostrukturen aus Metall, die im Nanosphere-Lithography-Verfahren aufgebracht werden und mit Detektormolekülen bestückt sind. Auch ein Vortrag mit dem Titel "Nanodetector for Collecting Rare Cells Out of the Circulating Blood", gegeben auf der Bioforum Konferenz 2009 in Lodz, offenbart funktionalisierte Biosensoren.

Viele Zelltypen, Moleküle, Tumor- und Biomarker sind in Körperflüssigkeiten zwar vorhanden, können aber oft wegen ihrer niedrigen Konzentration nicht effizient genug durch die herkömmlichen Methoden der Anreicherung gewonnen werden, um anschließend in etablierten diagnostischen Verfahren der klinischen Chemie, Pathologie oder Zytologie genutzt werden zu können.

Zum Beispiel ist das Anreichern von speziellen Zellen, insbesondere zirkulierenden Tumorzellen, aus einer Blutprobe (in vitro) mittels kommerziell erhältlicher paramagnetischer Nanopartikel und/oder Dichtegradientenzentrifugation möglich, jedoch in nur sehr begrenzter Anzahl und mit dem Nachteil, dass die Nanopartikel an oder in der Zelle binden und diese damit schädigen bzw. die Diagnostik erschweren können. Eine dieser kommerziellen Methoden spiegelt sich in einem Test wieder, in dem z.B. zirkulierende Tumorzellen aus 7,5 ml Blutvolumen mittels paramagnetischer Nanopartikel angereichert werden, um dann Aussagen über den Krankheitsverlauf machen zu können.

Der limitierende Faktor dieser Methode ist das gewonnene Probevolumen, welches beim Anwenden einer Detektionsvorrichtung zur in-vivo Anreicherung von Probenmaterial, z. B. eines funktionalisierten Katheter, um ein Vielfaches höher ist. Gefäßkatheter für die Anwendung medizinischer Interventionen sind meist zylinderförmig konstruiert. Der Vorteil dieser Form ist der relativ geringe Reibungswiderstand. Dennoch besteht bei dieser Form die Gefahr, dass der Blutstrom in kleineren Blutgefäßen verengt wird und dies zur Ausbildung einer Thrombose führt. Der Erfindung liegt die Aufgabe zugrunde, die Anreicherung von Probematerial unter Verwendung einer Detektionsvorrichtung der eingangs genannten Art weiter zu verbessern.

Um die der Erfindung zugrunde liegende Aufgabe zu lösen, wird die Detektionsvorrichtung zur in vivo und/oder in vitro Anreicherung von Probenmaterial nach Anspruch 1 bereit gestellt, umfassend ein biokompatibles Polymer und eine mit Detektionsrezeptoren bestückte Funktionsfläche, wobei das biokompatible Polymer die Funktionsfläche bildet und die Funktionsfläche eine dreidimensionale Struktur mit einander zugewandten Funktionsabschnitten aufweist, die von einer Probenflüssigkeit durchsetzbare Zwischenräume bilden.

Funktionsabschnitte gelten als einander zugewandt, wenn sie einen Winkel von kleiner als 180 Grad einschließen, sich gegenseitig also "sehen" können. Dadurch weist die Detektionsvorrichtung gegenüber einer herkömmlichen Detektionsvorrichtung mit einer glatten bzw. zylindrischen Funktionsfläche eine größere Funktionsfläche auf. Ferner kann die Probenflüssigkeit in den Zwischenräumen ideal über die Funktionsfläche geleitet werden und an den Detektionsrezeptoren angereichert werden. Zudem können die an den Detektionsrezeptoren andockenden Liganden in den Zwischenräumen festgehalten und so vor Abrieb besser geschützt werden. Vorzugsweise kann die Funktionsfläche auch ein gewisses Volumen an Probenflüssigkeit speichern. Die einander zugewandten Funktionsabschnitte können mit chemisch identischen oder chemisch verschiedenen Detektionsrezeptoren bestückt sein. Somit können bei einer Anwendung nach Bedarf auch verschiedene Liganden angereichert werden. Im Sinne dieser Erfindung werden alle Strukturen, insbesondere Rezeptoren oder Liganden, die geeignet sind, Zielmoleküle und Zielzellen einzufangen, als Detektionsrezeptoren bezeichnet. Ferner werden alle Zielmoleküle und Zielzellen, die an den Detektionsrezeptoren andocken können, vereinfachend als Liganden bezeichnet. Der Begriff Probenflüssigkeit bezeichnet eine in flüssiger Form vorliegende Probe.

Bevorzugte Weiterbildungen der Erfindung sind Gegenstände der Unteransprüche.

Es kann sich als hilfreich erweisen, wenn die Funktionsfläche im makroskopischen und/oder mikroskopischen Bereich dreidimensional strukturiert ist. Durch eine im makroskopischen bzw. sichtbaren Bereich dreidimensional strukturierte Funktionsfläche, die bspw. durch die sichtbare Geometrie der Detektionsvorrichtung bestimmt ist, kann die Probenflüssigkeit in vorteilhafter Weise über die Funktionsfläche geführt werden. Durch eine im mikroskopischen Bereich dreidimensional strukturierte Funktionsfläche kann die Strömungsgeschwindigkeit im Bereich der Grenzschicht verringert werden. Die Zwischenräume sind vorzugsweise mindestens derart dimensioniert, dass spezifische Liganden an den Detektionsrezeptoren andocken können. Vorzugsweise sind die Zwischenräume derart dimensioniert, dass in etwa die Anzahl an spezifischen Liganden aufgenommen und angeordnet werden können, die der Anzahl der Detektionsrezeptoren an den einander zugewandten Funktionsabschnitten entspricht. Dadurch wird die Funktionsfläche in idealer Weise genutzt. Ferner können die Liganden vor Abrieb noch besser geschützt werden.

In einer vorteilhaften Ausführung der Erfindung ist der Zwischenraum wenigstens abschnittsweise kanalförmig ausgebildet, wobei mehrere Zwischenräume vorzugsweise ein verzweigtes Netz von Kanälen bilden. Der Kanal kann sich über die gesamte Länge der Funktionsfläche erstrecken. Dadurch kann Probenflüssigkeit ideal zu den Detektionsrezeptoren geleitet werden. Durch die Auslegung der Größe der Zwischenräume kann eine Fließgeschwindigkeit und Fließrichtung der Probenflüssigkeit beeinflusst werden. Vorzugsweise erstreckt sich der Kanal zumindest abschnittsweise in Längsrichtung der Detektionsvorrichtung, so dass durch den Kanal eine natürliche Fließrichtung der Probenflüssigkeit möglichst wenig beeinträchtigt wird.

Es kann von Vorteil sein, wenn die Funktionsfläche mit Erhöhungen, Vertiefungen und/oder Verzweigungen ausgebildet ist, und/oder zumindest teilweise eine spiralförmige, schraubenförmige, schneckenförmige, wellenförmige, helikale, filamentöse, bürstenförmige, kammförmige, netzförmige, poröse, schwammförmige oder dergleichen Struktur umfasst. Derartige Formen besitzen große Oberflächen und sind für die Anwendung in der erfindungsgemäßen Detektionsvorrichtung gut geeignet. Spiralförmige, schraubenförmige, schneckenförmige, wellenförmige, helikale Strukturen weisen i.d.R. einen geringen Reibungswiderstand auf. Dadurch wird die Strömung der Probenflüssigkeit, bspw. Blut in einem Blutgefäß, wenig beeinträchtigt, selbst wenn Teile der Funktionsfläche an den Gefäßwänden anliegen. Filamentöse, bürstenförmige, kammförmige, netzförmige, poröse und schwammförmige Strukturen verlangsamen die natürliche Strömung der Probenflüssigkeit und eignen sich hervorragend als Flüssigkeitsspeicher, wodurch die Anreicherung der Liganden an den Detektionsrezeptoren zusätzlich begünstigt wird.

Es kann sich als vorteilhaft erweisen, wenn die Detektionsrezeptoren Antikörper, Antikörperfragmente, Aminosäuren-Strukturen, Nukleinsäure-Strukturen und/oder synthetische Strukturen mit spezifischer Affinität zu Zelloberflächen umfassen, vorzugsweise monoklonale Antikörper murinen Ursprungs, chimäre Antikörper oder humanisierte Antikörper, bevorzugt HLA-G und/oder EpCAM Antikörper.

Es kann auch praktisch sein, wenn die Funktionsfläche gesättigte Atomgruppen und kovalent gebundene Liganden und Rezeptoren umfasst, um unerwünschte Wechselwirkungen mit Blutbestandteilen und die Anbindung von unspezifischen Zellen und Molekülen zu verhindern.

Es kann weiter von Vorteil sein, wenn die Detektionsvorrichtung zumindest abschnittsweise als Führungsdraht, Stent und/oder Katheter ausgebildet ist. Derartige Komponenten sind kostengünstig verfügbar und können zur Verwirklichung der Erfindung modifiziert werden.

Es kann sich als praktisch erweisen, wenn die Detektionsvorrichtung einen mit der Funktionsfläche versehenen funktionalisierten Abschnitt sowie einen nicht funktionalisierten Abschnitt umfasst, die beide zu einem Stilett zusammengefügt sind. Dadurch kann die Handhabung der Detektionsvorrichtung erleichtert werden. Es kann vor Vorteil sein, wenn der funktionalisierte Abschnitt und der nicht funktionalisierte Abschnitt lösbar verbunden sind. So kann der funktionalisierte Abschnitt für die Zeitdauer, für welche die Detektionsvorrichtung bspw. in einen Blutkreislauf eingebracht wird, von dem nicht funktionalisierten Abschnitt getrennt. Bspw. wird der funktionalisierte Abschnitt an einer Venenverweilkanüle fixiert und später wieder mit dem nicht funktionalisierten Abschnitt gekoppelt.

Es kann aber auch praktisch sein, wenn der nicht funktionalisierte Abschnitt eine Markierung zur Applikationskontrolle und/oder ein abgerundetes Ende als Verletzungsschutz aufweist. Dadurch kann die Detektionsvorrichtung besser kontrolliert werden. Überdies wird die Verletzungs- und Infektionsgefahr bei der Anwendung der Detektionsvorrichtung verringert.

In einer vorteilhaften Ausführung der Erfindung weist die Detektionsvorrichtung einen Träger auf, der vorzugsweise wenigstens eine der folgenden Anforderungen erfüllt:
- Die Oberfläche des Trägers ist mit Erhöhungen, Vertiefungen und/oder Verzweigungen ausgebildet, und/oder umfasst zumindest teilweise eine spiralförmige, schraubenförmige, schneckenförmige, wellenförmige, helikale, filamentöse, bürstenförmige, kammförmige, netzförmige, poröse, schwammförmige oder dergleichen Struktur. Durch derartige Strukturen lassen sich die oben genannten Vorteile erzielen. Die Zwischenräume an der Oberfläche des Trägers, die bspw. durch die Porengröße oder die Dicke und Menge der Filamente bestimmt werden, sind derart, dass die spezifischen Liganden eindringen und an die Detektionsrezeptoren andocken können. Die Form und Gestaltung der Oberfläche des Trägers ist unabhängig von der Form und Gestaltung eines Substrats des Trägers an sich. Die Oberfläche des Trägers kann im makroskopischen und/oder im mikroskopischen Bereich dreidimensional strukturiert sein.
- Der Träger umfasst ein Substrat, das mit Erhöhungen, Vertiefungen und/oder Verzweigungen ausgebildet ist, und/oder zumindest teilweise eine spiralförmige, schraubenförmige, schneckenförmige, wellenförmige, helikale, filamentöse, bürstenförmige, kammförmige, netzförmige, poröse oder schwammförmige Struktur umfasst. Durch derartige Strukturen lassen sich die oben genannten Vorteile erzielen. Ein spiralförmiges Substrat umfasst vorzugsweise eine Litze aus einem oder mehreren Metalldrähten. Ein bürstenförmiges Substrat ist vorzugsweise durch einen oder mehrere Metall-, Glas-, Teflon- oder Kunststoff-Polymerfäden gebildet. Ein schraubenförmiges Substrat weist beispielsweise die Form eines Korkenziehers auf. Da das Substrat beschichtet werden kann, ist die Form und Gestaltung der Oberfläche des Trägers unabhängig von der Form und Gestaltung des Substrats. Das Substrat kann im makroskopischen und/oder im mikroskopischen Bereich dreidimensional strukturiert sein.

- Der Träger umfasst eine Beschichtung aus Metall, vorzugsweise aus einem Metall aus der 10. oder 11. Gruppe des Periodensystems der Elemente, bevorzugt aus Nickel, Kupfer, Palladium, Silber, Platin und/oder Gold.
- Der Träger umfasst funktionelle Gruppen, vorzugsweise organische funktionelle Gruppen, bevorzugt schwefel- und/oder stickstoffhaltige Verbindungen, besonders bevorzugt eine Beschichtung mit funktionellen Gruppen.
- Der Träger umfasst einen biokompatiblen Farbstoff, vorzugsweise eine Beschichtung aus einem biokompatiblen Farbstoff, um Eigenfluoreszenz während mikroskopischer Auswertung zu verringern.
- Der Träger ist als massiver Körper oder als Hohlkörper ausgebildet. Als Hohlkörper ausgebildet kann der Träger wenigstens einen Hohlraum und eine mit dem Hohlraum kommunizierende Öffnung aufweisen, über welche die Probenflüssigkeit in den Hohlraum gelangt. Die Funktionsfläche kann innerhalb des Hohlraums liegen. Eine derartige Detektionsvorrichtung eignet sich insbesondere für in vitro Anwendungen und dient bspw. gleichzeitig als Speichermedium für die Probeflüssigkeit, z. B. zum Transport.
- Der Träger ist aus einem biokompatiblen Material hergestellt. Dadurch kann eine Abwehrreaktion des Körpers bei der in vivo Anwendung der Detektionsvorrichtung verhindert werden.
- Der Träger ist zumindest teilweise aus Metall, vorzugsweise Edelstahl, medizinischem Edelstahl oder Titan; aus Glas, vorzugsweise Glasfaser; aus Kunststoff, vorzugsweise einem geschäumten Kunststoff, einem Polymer, bevorzugt Polyethylen,
   Polypropylen, Polyurethan, Polytetrafluorethylen, einem auf organischen Polymeren basierenden Kunststoff oder einer Kombination dieser Materialien gefertigt. Derartige Materialien sind i.d.R. kostengünstig verfügbar, flexibel und leicht formbar.
- Die Beschichtung ist mittels eines galvanischen Verfahrens auf den Träger aufgebracht. Diese Verfahren erweisen sich aus fertigungstechnischen Aspekten als vorteilhaft. Die Dicke der Beschichtung liegt vorzugsweise im Bereich von 0,1 bis 10 µm, bevorzugt im Bereich von 0,2 bis 5 µm, besonders bevorzugt im Bereich von 0,5 bis 1 µm.
- Der Träger umfasst gesättigte Atomgruppen und kovalent gebundene Liganden und Rezeptoren, um unerwünschte Wechselwirkungen mit Blutbestandteilen und die Anbindung von unspezifischen Zellen und Molekülen zu verhindern.
- Der Träger umfasst bzw. bildet die Funktionsfläche. Die Funktionsfläche befindet sich vorzugsweise an der Oberfläche des Trägers, wobei der Träger die einander zugewandten Funktionsabschnitte aufweist und direkt und mit den Detektionsrezeptoren bestückt ist. Dazu ist es vorteilhaft, wenn der Träger aus einem biokompatiblen Polymer besteht.

In einer anderen vorteilhaften Ausführung erfüllt das biokompatibles Polymer wenigstens eine der folgenden Anforderungen:
- Das biokompatible Polymer ist als zusammenhängende Polymerschicht ausgebildet. Dadurch kann die gesamte Oberfläche des Trägers durch die Polymerschicht abgedeckt oder abgeschirmt werden. Die Dicke der Polymerschicht liegt vorzugsweise im Bereich von 0,1 bis 10 µm, bevorzugt im Bereich von 0,5 bis 5 µm, besonders bevorzugt im Bereich von 1 bis 2 µm.
- Das biokompatible Polymer weist eine dreidimensionale, vorzugsweise filamentöse und/oder poröse Struktur auf. Diese Struktur bildet viele Zwischenräume, welche durch die an den Detektionsrezeptoren andockenden Liganden im Wesentlichen gefüllt werden, so dass die Liganden vor Abrieb noch besser geschützt sind. Das biokompatible Polymer kann im makroskopischen und/oder im mikroskopischen Bereich dreidimensional strukturiert sein.
- Das biokompatible Polymer weist eine dreidimensionale, vorzugsweise filamentöse, und/oder poröse Oberfläche auf. Die Oberfläche des biokompatiblen Polymers kann im makroskopischen und/oder mikroskopischen Bereich dreidimensional strukturiert sein.
- Das biokompatible Polymer weist eine kohlenstoffhaltige, verzweigte Molekülstruktur auf. Diese Molekülstruktur eignet sich hervorragend für die Anbindung der Detektionsrezeptoren und die Anreicherung von Liganden an den Detektionsrezeptoren. Dabei bildet diese Molekülstruktur eine filamentöse Funktionsfläche im Sinne der Erfindung, die im mikroskopischen Bereich dreidimensional strukturiert ist. Die verzweigten Molekülstrukturen umfassen zahlreiche Zwischenräume, die von gegenüberstehenden und mit Detektionsrezeptoren bestückten Funktionsabschnitten in Form von Polymermolekülen gebildet sind. Diese Zwischenräume sind von einer Probenflüssigkeit durchsetzbar und bilden einen Flüssigkeitsspeicher, wodurch die Anreicherung der Liganden an den Detektionsrezeptoren besonders begünstigt wird. Im Bereich der Grenzschicht an einer mit dieser Molekülstruktur besetzten Oberfläche wird die Umströmung von einer Probenflüssigkeit erheblich verlangsamt. Dadurch wird die Anreicherung der Liganden zusätzlich begünstigt.
- Das biokompatible Polymer ist vorzugsweise über funktionelle Gruppen mit dem Träger wirkverbunden, bevorzugt durch chemische Bindung, besonders bevorzugt durch kovalente Bindung.
- Das biokompatible Polymer umfasst funktionelle Gruppen, vorzugsweise Carboxyl-Gruppen, wobei die funktionellen Gruppen vorzugsweise durch chemische Aktivierung eine unausgeglichene Molekülladung aufweisen, wobei die funktionellen Gruppen bevorzugt auf die Detektionsrezeptoren abgestimmt sind.
- Das biokompatible Polymer weist hydrophile Eigenschaften auf und ist vorzugsweise ein Hydrogel.
- Das biokompatible Polymer umfasst chemisch und/oder enzymatisch spaltbare Gruppen, welche die Ablösung der Liganden erleichtert. Die chemisch und/oder enzymatisch spaltbaren Gruppen sind vorzugsweise die funktionellen Gruppen, an welchen die Detektionsrezeptoren gebunden sind.
- Das biokompatible Polymer umfasst gesättigte Atomgruppen und kovalent gebundene Detektionsrezeptoren, um unerwünschte Wechselwirkungen mit Blutbestandteilen und die Anbindung von unspezifischen Zellen und Molekülen zu verhindern.
- Das biokompatible Polymer ist in einem Hohlraum des Trägers angeordnet. Dadurch kann ein Hohlraum des Trägers zur Anreicherung von Liganden genutzt werden. Dort sind die Liganden vor Abrieb optimal geschützt.
- Das biokompatible Polymer ist vernetzt.
- Das biokompatible Polymer umfasst die Funktionsfläche. Die Funktionsfläche befindet sich vorzugsweise an der Oberfläche des biokompatiblen Polymers. Das biokompatible Polymer kann direkt mit den Detektionsrezeptoren bestückt sein.

In noch einer anderen vorteilhaften Ausführung der Erfindung ist die Funktionsfläche mit einer Schutzschicht überzogen, wobei die Schutzschicht vorzugsweise wenigstens eine der folgenden Anforderungen erfüllt:
- Die Schutzschicht ist in Flüssigkeiten, insbesondere in Körperflüssigkeiten, vorzugsweise in Blut, löslich. Dadurch kann die Funktionsfläche automatisch freigelegt werden, sobald die Schutzschicht in Kontakt mit der Probenflüssigkeit gelangt.
- Die Schutzschicht ist biokompatibel. Dadurch werden Abwehrreaktionen des Körpers bei der in vivo Anwendung der Detektionsvorrichtung weitgehend unterbunden.
- Die Schutzschicht ist organisch kristallin und umfasst wenigstens einen der folgenden Bestandteile: Alginate, vorzugsweise hochreine Alginate, Polyethylenglykole, zyklische und nicht zyklische Oligosaccharide, Polysaccharide, antioxidative Aminosäuren, Proteine oder Vitamine. Derartige Bestandteile sind biokompatibel und leicht löslich.

Es kann nützlich sein, wenn die Detektionsrezeptoren vorzugsweise über Linker oder organische funktionelle Gruppen mit der Funktionsfläche, vorzugsweise mit dem Träger und/oder dem biokompatiblen Polymer, wirkverbunden sind, vorzugsweise durch chemische Bindung, bevorzugt durch kovalente Bindung. Dadurch kann eine unspezifische Adsorption an der Funktionsfläche weitgehend verhindert werden.

Ein weiterer unabhängiger Aspekt der Erfindung betrifft eine Detektionsvorrichtung nach wenigstens einer der vorangegangenen Ausführungen, hergestellt durch:
- Bereitstellung eines Trägers,
- chemische Aktivierung des Trägers durch chemische, vorzugsweise kovalente Bindung der Detektionsrezeptoren direkt oder über ein biokompatibles Polymer an funktionelle Gruppen des Trägers, vorzugsweise an organische funktionelle Gruppen des Trägers, bevorzugt über schwefel- und/oder stickstoffhaltige Verbindungen.

Die Detektionsvorrichtung kann jedes der zuvor genannten Merkmale aufweisen.

Noch ein weiterer unabhängiger Aspekt berifft die Verwendung einer Detektionsvorrichtung nach einer der vorangegangenen Ausführungen zur invasiven Anreicherung von Probenmaterial, zur Elimination von Arzneimittel, zur Elimination von radioaktiven Tracern, zur Elimination von Magnetobeads, zur Gewinnung von Tumor- oder Biomarkern und/oder zur Elimination von Toxinen, oder zur Anreicherung von Zellen, umfassend embryonale Trophoblasten, disseminierten Tumorzellen, insbesondere von hämatogen metastasierenden Tumoren. Dadurch lassen sich die genannten Vorteile erzielen.

Noch ein weiterer unabhängiger Aspekt betrifft ein Verfahren zur Anreicherung von Probenmaterial unter Benutzung einer Detektionsvorrichtung nach wenigstens einer der vorangegangenen Ausführungen, wobei das Verfahren die folgenden Schritte umfasst:
- Beaufschlagen der Funktionsfläche mit einer Probenflüssigkeit,
- Anreicherung von Probenmaterial, vorzugsweise Zellen, DNA, RNA, Proteine, Peptide, synthetische Moleküle, an den Detektionsrezeptoren und
- Entfernung der Probenflüssigkeit.

Die Detektionsvorrichtung kann jedes der zuvor genannten Merkmale aufweisen. Bevorzugte Weiterbildungen der Erfindung ergeben sich durch Kombinationen der Unteransprüche oder der darin genannten Teilmerkmale.

### Kurze Beschreibung der Figuren

- Figur 1: ist eine schematische Darstellung einer erfindungsgemäßen Detektionsvorrichtung in Form eines Stiletts.
- Figur 2: zeigt den Schichtaufbau eines funktionalisierten Teils der Detektionsvorrichtung.
- Figur 3: ist eine schematische Darstellung eines schraubenförmigen Trägers.
- Figur 4: ist eine Querschnittsansicht eines bürstenförmigen Trägers.
- Figur 5: ist eine schematische Darstellung eines helikalen Trägers.

### Detaillierte Beschreibung der bevorzugten Ausführungsbeispiele

Die bevorzugten Ausführungsbeispiele der Erfindung werden nachstehend mit Bezug auf die beigefügten Zeichnungen beschrieben.

### Erstes Ausführungsbeispiel

Die erfindungsgemäße Detektionsvorrichtung 1 nach dem ersten Ausführungsbeispiel ist biofunktionalisierter, medizinischer Detektionskatheter für die invasive (in vivo) Anreicherung von seltenen Zellen, Biomolekülen oder Arzneimitteln. Ein derartiger Detektionskatheter wird auch als medizinscher Nano-Katheter (MN-K) bzw. medical nano-catheter (MN-C) bezeichnet.

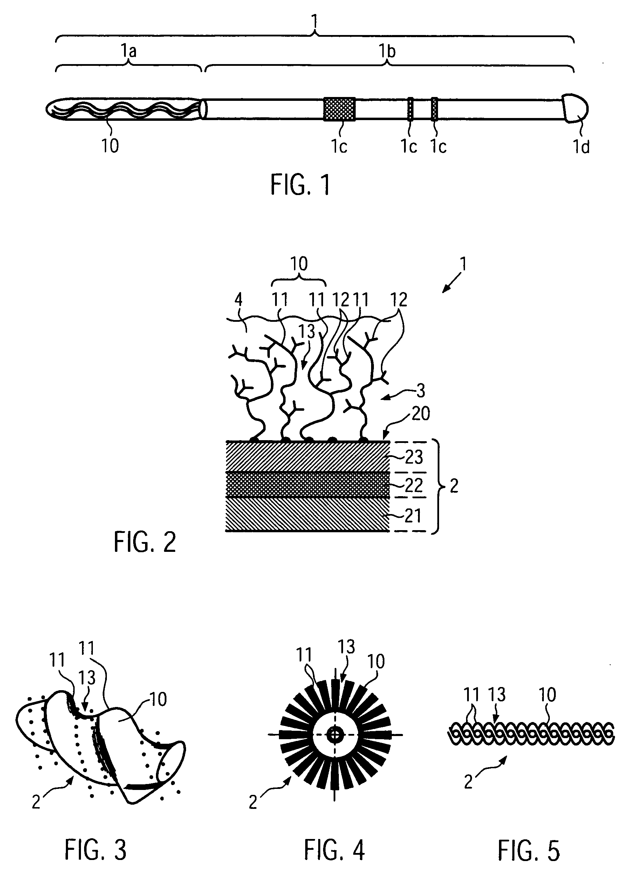

Figur 1 ist eine schematische Darstellung des Detektionskatheters. Der funktionalisierte Teil 1 a ist ca. 2 cm lang und umfasst die mit Detektionsrezeptoren bestückte Funktionsfläche 10. Der nicht funktionalisierte Teil 1 b ist ca. 14 cm lang und umfasst Markierungen 1 c für die Applikationskontrolle sowie am Ende eine Halbkugel 1 d als Verletzungsschutz für den Anwender. Der funktionalisierte Teil 1 a sowie der nicht funktionalisierte Teil 1 b des Katheters 1 sind zu einem Stilett zusammengefügt.

Figur 2 zeigt den Schichtaufbau des funktionalisierten Teils 1a des Detektionskatheters.

Der Träger 2 umfasst bspw. eine schraubenförmige Struktur, wie in Figur 3 dargestellt. Der Schraubengang des Trägers 2 bildet im makroskopischen bzw. sichtbaren Bereich eine dreidimensional strukturierte Funktionsfläche 10 mit einander zugewandten Funktionsabschnitten 11 und einen von einer Probenflüssigkeit durchsetzbarem Zwischenraum 13. Nach dem Prinzip eines Stents könnten spiralisierte Drähte an den Durchmesser eines Blutgefäßes angepasst zu werden, in dem die temporäre Platzierung erfolgen soll. Die spiralisierte Form ermöglicht anders als ein zylindrischer Katheter 1 eine weniger starke Verengung des Blutgefäßes. Die Vertiefungen oder Schlucktiefen verhindern, dass die detektierten Moleküle oder Zellen (angedeutet durch punktierte Linien) durch die Entnahme nicht abgerieben werden.

Die Form des Katheters 1 kann beliebig gestaltet werden und ist abhängig von der Anwendung. Der Katheter 1 kann beispielsweise auch einen helikalen oder bürstenförmigen Träger 2 gemäß den Figuren 4 oder 5 aufweisen. Der bürstenförmige Träger 2 gemäß Figur 4 kann aus einem oder mehreren Metall-, Glas-, Teflon- oder Kunststoff-Polymerfäden konstruiert sein, wobei die Fäden im makroskopischen bzw. sichtbaren Bereich eine dreidimensional strukturierte Funktionsfläche 10 mit einander zugewandten Funktionsabschnitten 11 und von Probenflüssigkeit durchsetzbaren Zwischenräumen 13 bilden. Der helikale Träger 2 gemäß Figur 5 kann aus einer Litze mit einem oder mehreren Metalldrähten bestehen. Eine im makroskopischen Bereich dreidimensional strukturierte Funktionsfläche 10 mit einander zugewandten Funktionsabschnitten 11 und von Probenflüssigkeit durchsetzbaren Zwischenräumen 13 wird in diesem Fall durch die Flanken der Helix gebildet.

Aufgrund der im sichtbaren Bereich dreidimensional strukturierten Funktionsfläche 10 ist Träger 2 in der Lage, Verengungen des Blutstroms zu verringern und durch die spezielle Geometrie den laminaren Blutfluss so zu verändern, dass die Wahrscheinlichkeit einer Anbindung von Zielmolekülen und Zielzellen im Vergleich zu einem zylinderförmigen Katheter um den Faktor 2 größer wird.

Ein Substrat 21 aus medizinischem Edelstahldraht mit einem Durchmesser von ca. 0,5 mm verleiht dem Träger 2 seine sichtbare Struktur. Das Substrat 21 kann eine oder mehrere Beschichtungen 22, 23 aufweisen. Vorzugsweise ist das Substrat 21 mit einer Goldbeschichtung 22 mit eine Dicke von 0,5 bis 1,0 µm überzogen, die durch galvanische Verfahren, keramische Verfahren, Zementation oder durch Bedampfung aufgebracht wird. Das Substrat 21 kann ferner mit einem biokompatiblen Farbstoff beschichtet sein, um Eigenfluoreszenz des Grundmaterials während mikroskopischer Auswertung zu verringern.

Die chemische Aktivierung des Trägers 2 erfolgt über eine Affinitätsreaktion meist durch schwefel- oder stickstoffhaltige Verbindungen, an denen wiederum direkt oder über Polymerketten spezifische Detektionsrezeptoren 12 gebunden werden können.

Vorzugsweise wird auf dem Träger 2 über nasschemische oder physikalische Verfahren eine kovalente Sekundärschicht bestehend aus einem funktionalen biokompatiblen Polymer 3 aufgetragen. Die Schichtdicke kann 1 bis 2 µm betragen. Dadurch wird der Träger 2 chemisch aktiviert. Durch die Oberflächenveredelung und chemische Aktivierung lassen sich spezifische Antikörper, insbesondere monoklonale Antikörper murinen Ursprungs, chimäre Antikörper, humanisierte Antikörper, oder Fragmente dieser Antikörper oder Aminosäure-Strukturen oder Nukleinsäure-Strukturen oder synthetische Strukturen mit spezifischer Affinität zu Zelloberflächen oder Molekülen als Detektionsrezeptoren 12 kovalent anbinden.

Gerade bei einer komplexen Probenflüssigkeit wie Blut ist nicht nur eine dauerhafte Anbindung der Detektionsrezeptoren 12 unter Erhalt der biologischen Funktion, sondern auch eine effiziente Unterdrückung unspezifischer Adsorptionsprozesse für die selektive Anbindung der Liganden von entscheidender Bedeutung.

Eine Zwischenschicht 23 hat hierbei die Aufgabe, eine effektive Abschirmung der Oberfläche des Substrats 21 zu gewährleisten und gleichzeitig die funktionellen Gruppen für die Bindung der biokompatiblen Polymerschicht 3 in ausreichender Dichte zur Verfügung zu stellen. Das Zwischenschichtsystem bildet demnach einen Haftvermittler zwischen der Goldbeschichtung 22 des Substrats 21 und der biokompatiblen Polymerschicht 3.

Das biokompatible Polymer 3 ist vorzugsweise ein Hydrogel mit kohlenstoffhaltigen langen verzweigten Makromolekülen, welche über eine hohe Anzahl an funktionellen Gruppen, z.B. Carboxyl-Gruppen bzw. Polycarboxylaten, verfügen. Die Art der funktionellen Gruppen richtet sich nach den Moleküleigenschaften der spezifischen Detektionsrezeptoren 12. Das biokompatible Hydrogel gewährleistet dadurch die dauerhafte kovalente Bindung der Detektionsrezeptoren 12 unter Erhalt der biologischen Funktion und verhindert gleichzeitig, dass die Detektionsrezeptoren 12 durch unspezifische Adsorptionsphänomene in ihrer Erkennungsfunktion beeinträchtigt werden. Hydrogele sind dreidimensional vernetzte hydrophile Polymere, die Flüssigkeiten wie z.B. Wasser aufnehmen, selbst aber darin unlöslich sind. Hauptbestandteile des Hydrogels sind Polyacrylsäure (PAA) und Polyethylenglycol (PEG). Über eine geeignete Auswahl der Monomerbausteine, des Vernetzungsgrades und der Vernetzungsdichte lassen sich Eigenschaftsprofile je nach gewünschten Anforderungen oder Einsatzbereiche maßschneidern. Eine wesentliche Eigenschaft ist die Biokompatibilität, d. h. die Verträglichkeit des Hydrogels mit dem lebenden Gewebe. Durch die verzweigten Polymerketten des biokompatiblen Polymers 3 wird aber auch die thrombogene Wirkung während der invasiven Anwendung unterbunden. Durch chemische Aktivierung erhalten die funktionellen Gruppen eine unausgeglichene Molekülladung, die es ermöglicht, gelöste Detektionsrezeptoren 12 aus einer Lösung elektrostatisch anzuziehen und kovalent zu binden. Die dauerhaft an der Polymerschicht 3 immobilisierten Detektionsrezeptoren 12 dienen der spezifischen Bindung der Liganden bzw. Zielmoleküle und Zielzellen über ihre Oberflächenantigene und ermöglichen so die Funktion der Detektionsvorrichtung 1. Zusätzlich können in diesem biokompatiblen Polymer 3 chemisch oder enzymatisch spaltbare Gruppen enthalten sein, um die quantitative Gewinnung von gebundenen Zielmolekülen oder Zellen zu vereinfachen.

Die verzweigten Molekülstrukturen des biokompatiblen Polymers 3 bilden eine im mikroskopischen Bereich dreidimensional strukturierte Funktionsfläche 10 mit einander zugewandten Funktionsabschnitten 11 und von Probenflüssigkeit durchsetzbaren Zwischenräumen 13, wie in Figur 1 schematisch dargestellt ist. Während die im makroskopisch bzw. sichtbaren Bereich dreidimensional strukturierte Oberfläche des Trägers 2 (vgl. Figuren 3, 4 und 5) die Probenflüssigkeit in vorteilhafter Weise über den funktionellen Teil 1 a des Katheters 1 leitet, verlangsamt die im mikroskopischen Bereich dreidimensional strukturierte Funktionsfläche 10 des biokompatiblen Polymers 3 (vgl. Figur 2) die Strömung der Probenflüssigkeit im Bereich der Grenzschicht und begünstigt die Anreicherung der Liganden an den Detektionsrezeptoren 12.

Zum Konservieren und zum Schutz vor den Bedingungen der Endsterilisation sowie zum Strahlenschutz und zur Haltbarkeit des Produkts wird über das biokompatible Polymer 3 eine biokompatible Schutzschicht (tertiäre Schicht bzw. Stabilisatorschicht) 4 aufgebracht. Diese Schutzschicht 4 trocknet über der Sekundärschicht ein und bildet ein dichtes Netz aus kristallinen Strukturen und stabilisiert und konserviert somit den funktionellen Teil 1a des Katheters 1. Die Schutzschicht 4 ist nicht kovalent gebunden. Im Blutstrom geht die Schutzschicht 4 in Lösung und gibt die Funktionsfläche 10 des Katheters frei. Alternativ kann die Schutzschicht 4 vor der Anwendung mit sterilem Wasser abgewaschen werden.

Die Schutzschicht 4 kann hochreine Alginate, Polyethylenglykole, zyklische und nicht zyklische Oligosaccharide und Polysaccharide, antioxidative Aminosäuren, Proteine und Vitamine umfassen. Die Schutzschicht 4 besteht vorzugsweise aus einem biokompatiblen hochviskosen Polysaccharid, welches als Medium für zugesetzte Aminosäuren, Proteinen, Vitaminen und stabilisierende Polysaccharide dient. Die hohe Viskosität erlaubt eine rasche Benetzbarkeit der Oberfläche. Die angelagerte Schutzschicht 4 klebt an der Sekundärbeschichtung und verhindert das Eindringen von Fremdsubstanzen während einer Lagerung.

Die zugesetzten Aminosäuren, Proteine und Vitamine sind im Vergleich zu den spezifischen Liganden in höheren Konzentrationen vorhanden und dadurch in der Lage, die Wahrscheinlichkeit einer Schädigung der Zielmoleküle durch radikale Moleküle oder Ladungsträger auf sich zu lenken bzw. abzufangen und durch Rekombinationsprozesse zerstörte chemische Bindungen wiederherzustellen.

Der fertig gestellte Katheter 1 wird in keimarmer Umgebung verpackt. Die Endsterilisation erfolgt mittels Gamma-Bestrahlung bei einer Strahlungsdosis von 25 kGy. Der Katheter 1 ist für die einmalige Anwendung vorgesehen.

### Anwendung der Detektionsvorrichtung

Der erfindungsgemäß hergestellte Katheter 1 mit veredelter Funktionsfläche 10 und mit gekoppelten Detektionsrezeptoren 1 eignet sich für die Gewinnung seltener Zellen aus dem Blutkreislauf. Hierzu zählen folgende Anwendungsbeispiele:
- Gewinnung von embryonalen Trophoblasten aus dem mütterlichen Blutkreislauf mit z.B. spezifischen Antikörperfragmenten (F(ab)-Fragmenten) und murinen monoklonalen Antikörpern (IgG), welche das für Trophoblasten typische Zelloberflächenprotein HLA-G erkennen können.
- Gewinnung von disseminierten Tumorzellen, insbesondere von hämatogen metastasierenden Tumoren z.B. mit dem humanisierten Antikörper Anti-EpCAM, welcher das für viele Krebszellen typische Zelloberflächenprotein EpCAM erkennt.

Eine bevorzugte Anwendung der Detektionsvorrichtung 1 liegt in der pränatalen und Krebs-Diagnostik. Die Detektionsvorrichtung 1 kann beispielsweise dafür eingesetzt werden, im Blutkreislauf von Schwangeren oder Krebspatienten zirkulierende fetale Zellen oder Tumorzellen zu isolieren. Zur Anwendung wird die Detektionsvorrichtung 1 über ein geeignetes kommerziell erhältliches Braunülensystem in die Vene eingeführt und in den venösen Blutkreislauf appliziert. Die Verweildauer in der Vene kann ca. 30 min betragen. Nach der Entnahme der Detektionsvorrichtung 1 aus der Blutbahn werden die auf der Detektionsvorrichtung 1 gebundenen Zellen mittels gezielter Labordiagnostik weiter angereichert und molekularbiologisch sowie zellbiologisch charakterisiert.

Ziel des durchzuführenden minimal invasiven Verfahrens ist die Selektion von fetalen oder Tumor-Zellen aus dem Blut. Auf Grund der niedrigen Zellkonzentration der Zellen im Blut wäre eine Blutentnahme von ca. 0,5 l notwendig, um die gewünschte Zielzellzahl zu erhalten. Dies ist aus medizinischer Sicht jedoch ausgeschlossen.

Bei der pränatalen Diagnostik soll eine eventuelle Chromosomenaberration (z. B. Trisomie 21 (Down-Syndrom)) mit Hilfe der im Blut der Mutter enthaltenen fetalen Zellen nachgewiesen werden. Das Down-Syndrom wird pränatal bislang sicher nur durch invasive Verfahren, die jeweils ein Abortrisiko von 1 % aufweisen, diagnostiziert: Chorionzottenbiopsie zwischen der 11. und 14. Schwangerschaftswoche und Amniozentese (Fruchtwasseruntersuchung) ab der 15. Schwangerschaftswoche. Das Verfahren hingegen, das ab der 9. Schwangerschaftswoche einsetzbar sein wird, weist kein Risiko für den Föten auf und kann im Erst-Trimester-Screening eingesetzt werden. Somit kann auf die Amniozentesen verzichten werden.

Fetale Trophoblastenzellen aus der Plazenta können ab der 6. Schwangerschaftswoche im Blutkreislauf der Mutter nachgewiesen werden. Es kommen nur ca. 2 - 5 dieser Zellen pro ml Mutterblut vor. Diese Trophoblastenzellen besitzen einen membrangebundenen HLA-G Komplex (Antigen), der an bestimmte Antikörper bindet. Vorzugsweise wird ein spezifischer HLA-G Antikörper als Detektionsrezeptor 12 eingesetzt, der nur mit membrangebundenem HLA-G (Antigen) reagiert und somit nur die gewünschten fetalen Zellen aus dem Mutterblut fangen soll.

Krebstumorzellen können mit dem EpCAM Antikörper (gegen das EpCAM Antigen) angereichert werden, der in seinen konstanten Domänen humanisiert ist und kovalent and das Hydrogel gebunden wird.

### Zweites Ausführungsbeispiel

Die erfindungsgemäße Detektionsvorrichtung nach dem zweiten Ausführungsbeispiel betrifft eine in vitro Detektionsvorrichtung, welche eine poröse oder filamentöse Funktionsfläche 10 aufweist und in der Lage ist, aus Körperflüssigkeiten oder aus anderem flüssigen Untersuchungsmaterial, Zellen oder Moleküle anzureichern. Für ähnliche Merkmale werden dieselben Bezugszeichen verwendet wie im ersten Ausführungsbeispiel und es wird auf obige Beschreibung Bezug genommen.

Die Detektionsvorrichtung 1 umfasst einen Träger 2 mit einem abgedichteten Hohlraum, durch welchen die Probeflüssigkeit geleitet wird. Der Hohlraum enthält eine poröse oder filamentöse Matrix, vorzugsweise ein biokompatibles Polymer 3 oder einen Polymerschaum. Die oben genannten Detektionsrezeptoren 12 können direkt oder über Linker kovalent an die Oberfläche dieser porösen oder filamentösen Matrix gebunden werden. Die Porenwände oder Filamente bilden im makroskopischen und/oder mikroskopischen Bereich eine dreidimensional strukturierte Funktionsfläche 10 mit einander zugewandten Funktionsabschnitten 11 und von Probenflüssigkeit durchsetzbaren Zwischenräumen 13. Die dadurch hervorgerufene Oberflächenvergrößerung erhöht die Anzahl der zur Verfügung stehenden Detektionsrezeptoren 12 und sorgt damit für eine verbesserte Anreicherung der Zielmoleküle oder Zielzellen. Die Porengröße oder die Dicke und Menge der Filamente der Matrix ist variabel und kann der Art der zu detektierenden Zielmoleküle oder Zielzellen angepasst werden.

Die Beschichtung der Detektionsvorrichtung 1 gestaltet sich analog zur Katheterbeschichtung nach dem ersten Ausführungsbeispiel mit dem Unterschied, dass der Träger 2 hauptsächlich aus biokompatiblen Kunststoffen, wie Polyurethan, Polyethylen, Teflon oder Polypropylen besteht. Der Träger 2 kann durch nasschemische oder physikalische Verfahren funktionalisiert werden. Die Funktionsfläche 10 der Matrix kann aktivierbare organische Polymere und funktionelle Gruppen umfassen.

Diese Detektionsvorrichtung eignet sich zur Anreicherung bzw. Aufkonzentrierung von Zellen oder Biomolekülen aus Körperflüssigkeiten oder anderen flüssigen Analyseproben. Die flüssigen Untersuchungsproben werden in die Detektionsvorrichtung 1 appliziert oder werden sofort mittels Luer-Lock Entnahme- bzw. Probenahmesysteme eingeleitet. Das isolierte und angereicherte Material kann sofort einer nachgeschalteten Diagnostik, beispielweise über eine Lab-on-Chip Technologie, zugeführt werden.

Diese Detektionsvorrichtung 1 zeichnet sich aufgrund der Konstruktion durch sehr einfache Handhabung aus, welche ohne großen Zeitaufwand mittels eines Kits in jeder diagnostischen oder klinischen Einrichtung verwendet werden kann.

## Patentansprüche

1. Detektionsvorrichtung (1) zur in vivo und/oder in vitro Anreicherung von Probenmaterial, umfassend ein biokompatibles Polymer (3) und eine mit Detektionsrezeptoren (12) bestückte Funktionsfläche (10), wobei das biokompatible Polymer (3) die Funktionsfläche bildet und die Funktionsfläche (10) eine dreidimensionale Struktur mit einander zugewandten Funktionsabschnitten (11) aufweist, die wenigstens einen von einer Probenflüssigkeit durchsetzbaren Zwischenraum (13) bilden, wobei die Detektionsvorrichtung (1) einen Träger (2) aufweist, **dadurch gekennzeichnet, dass** der Träger (2) eine Beschichtung (22) aus Metall aufweist, die mittels eines galvanischen Verfahrens auf den Träger (2) aufgebracht ist, und dass das biokompatible Polymer (3) gesättigte Atomgruppen und kovalent gebundene Detektionsrezeptoren (12) aufweist.

2. Detektionsvorrichtung (1) nach Anspruch 1, **dadurch gekennzeichnet, dass** die Funktionsfläche (10) im makroskopischen und/oder im mikroskopischen Bereich dreidimensional strukturiert ist.

3. Detektionsvorrichtung (1) nach wenigstens einem der vorangegangenen Ansprüche, **dadurch gekennzeichnet, dass** der Zwischenraum (13) wenigstens abschnittsweise kanalförmig ausgebildet ist, wobei mehrere Zwischenräume vorzugsweise ein verzweigtes Netz von Kanälen bilden.

4. Detektionsvorrichtung (1) nach wenigstens einem der vorangegangenen Ansprüche, **dadurch gekennzeichnet, dass** die Funktionsfläche (10) mit Erhöhungen, Vertiefungen und/oder Verzweigungen ausgebildet ist, und/oder zumindest teilweise eine spiralförmige, schraubenförmige, schneckenförmige, wellenförmige, helikale, filamentöse, bürstenförmige, kammförmige, netzförmige, poröse, oder schwammförmige Struktur umfasst.

5. Detektionsvorrichtung (1) nach wenigstens einem der vorangegangenen Ansprüche, **dadurch gekennzeichnet, dass** die Detektionsrezeptoren (12) Antikörper, Antikörperfragmente, Aminosäuren-Strukturen, Nukleinsäure-Strukturen und/oder synthetische Strukturen mit spezifischer Affinität zu Zelloberflächen umfassen, vorzugsweise monoklonale Antikörper murinen Ursprungs, chimäre Antikörper oder humanisierte Antikörper, bevorzugt HLA-G und/oder EpCAM Antikörper.

6. Detektionsvorrichtung (1) nach wenigstens einem der vorangegangenen Ansprüche, **dadurch gekennzeichnet, dass** die Detektionsvorrichtung (1) zumindest abschnittsweise als Führungsdraht, Stent und/oder Katheter ausgebildet ist.

7. Detektionsvorrichtung (1) nach wenigstens einem der vorangegangenen Ansprüche, **dadurch gekennzeichnet, dass** die Detektionsvorrichtung (1) einen mit der Funktionsfläche (10) versehenen funktionalisierten Abschnitt (1 a) sowie einen nicht funktionalisierten Abschnitt (1 b) umfasst, die beide zu einem Stilett zusammengefügt sind.

8. Detektionsvorrichtung (1) nach Anspruch 7, **dadurch gekennzeichnet, dass** der nicht funktionalisierte Abschnitt (1b) eine Markierung (1c) zur Applikationskontrolle und/oder ein abgerundetes Ende (1d) als Verletzungsschutz aufweist.

9. Detektionsvorrichtung (1) nach wenigstens einem der vorangegangenen Ansprüche, **dadurch gekennzeichnet, dass** der Träger (2) wenigstens eine der folgenden Anforderungen erfüllt:
a. Die Oberfläche (20) des Trägers (2) ist mit Erhöhungen, Vertiefungen und/oder Verzweigungen ausgebildet, und/oder umfasst zumindest teilweise eine spiralförmige, schraubenförmige, schneckenförmige, wellenförmige, helikale, filamentöse, bürstenförmige, kammförmige, netzförmige, poröse, schwammförmige oder dergleichen Struktur.
b. Der Träger (2) umfasst ein Substrat (21), das mit Erhöhungen, Vertiefungen und/oder Verzweigungen ausgebildet ist, und/oder zumindest teilweise eine spiralförmige, schraubenförmige, schneckenförmige, wellenförmige, helikale, filamentöse, bürstenförmige, kammförmige, netzförmige, poröse, schwammförmige oder dergleichen Struktur umfasst.
c. Der Träger (2) umfasst eine Beschichtung (22) aus einem Metall aus der 10. oder 11. Gruppe des Periodensystems der Elemente, bevorzugt aus Nickel, Kupfer, Palladium, Silber, Platin und/oder Gold.
d. Der Träger (2) umfasst funktionelle Gruppen, vorzugsweise organische funktionellen Gruppen, bevorzugt schwefel- und/oder stickstoffhaltige funktionelle Gruppen, besonders bevorzugt eine Beschichtung (23) mit funktionellen Gruppen.
e. Der Träger (2) umfasst einen biokompatiblen Farbstoff, vorzugsweise eine Beschichtung aus einem biokompatiblen Farbstoff.
f. Der Träger (2) ist als massiver Körper oder als Hohlkörper ausgebildet.
g. Der Träger (2) ist aus einem biokompatiblen Material hergestellt.
h. Der Träger (2) ist zumindest teilweise aus Metall, vorzugsweise Edelstahl, medizinischem Edelstahl oder Titan; aus Glas, vorzugsweise Glasfaser; aus Kunststoff, vorzugsweise einem geschäumten Kunststoff, einem Polymer, bevorzugt Polyethylen, Polypropylen, Polyurethan, Polytetrafluorethylen, einem auf organischen Polymeren basierenden Kunststoff oder einer Kombination dieser Materialien gefertigt.
i. Der Träger (2) umfasst gesättigte Atomgruppen und kovalent gebundene Liganden und Rezeptoren.
j. Der Träger (2) umfasst und/oder bildet die Funktionsfläche (10).

10. Detektionsvorrichtung (1) nach wenigstens einem der vorangegangenen Ansprüche, **dadurch gekennzeichnet, dass** das biokompatibles Polymer (3) wenigstens eine der folgenden Anforderungen erfüllt:
a. Das biokompatible Polymer (3) ist als zusammenhängende Polymerschicht ausgebildet.
b. Das biokompatible Polymer (3) weist eine dreidimensionale, vorzugsweise filamentöse und/oder poröse Struktur auf.
c. Das biokompatible Polymer (3) weist eine dreidimensionale, vorzugsweise filamentöse, und/oder poröse Oberfläche auf.
d. Das biokompatible Polymer (3) weist eine kohlenstoffhaltige, verzweigte Molekülstruktur auf.
e. Das biokompatible Polymer (3) ist vorzugsweise über funktionelle Gruppen mit dem Träger (2) wirkverbunden, bevorzugt durch chemische Bindung, besonders bevorzugt durch kovalente Bindung.
f. Das biokompatible Polymer (3) umfasst funktionelle Gruppen, vorzugsweise Carboxyl-Gruppen, wobei die funktionellen Gruppen vorzugsweise durch chemische Aktivierung eine unausgeglichene Molekülladung aufweisen, wobei die funktionellen Gruppen bevorzugt auf die Detektionsrezeptoren (12) abgestimmt sind.
g. Das biokompatible Polymer (3) weist hydrophile Eigenschaften auf und ist vorzugsweise ein Hydrogel.
h. Das biokompatible Polymer (3) umfasst chemisch und/oder enzymatisch spaltbare Gruppen.
i. Das biokompatible Polymer (3) ist in einem Hohlraum des Trägers (2) angeordnet.
j. Das biokompatible Polymer (3) ist vernetzt.
k. Das biokompatible Polymer (3) umfasst die Funktionsfläche (10).

11. Detektionsvorrichtung (1) nach wenigstens einem der vorangegangenen Ansprüche, **dadurch gekennzeichnet, dass** die Funktionsfläche (10) mit einer Schutzschicht (4) überzogen ist, wobei die Schutzschicht (4) vorzugsweise wenigstens eine der folgenden Anforderungen erfüllt:
a. Die Schutzschicht (4) ist in Flüssigkeiten, insbesondere in Körperflüssigkeiten, vorzugsweise in Blut, löslich.
b. Die Schutzschicht (4) ist biokompatibel.
c. Die Schutzschicht (4) ist organisch kristallin und umfasst wenigstens einen der folgenden Bestandteile: Alginate, vorzugsweise hochreine Alginate, Polyethylenglykole, zyklische und nicht zyklische Oligosaccharide, Polysaccharide, antioxidative Aminosäuren, Proteine oder Vitamine.

12. Detektionsvorrichtung (1) nach wenigstens einem der vorangegangenen Ansprüche, **dadurch gekennzeichnet, dass** die Detektionsrezeptoren (12) vorzugsweise über Linker oder organische funktionelle Gruppen mit der Funktionsfläche (10), vorzugsweise mit dem Träger (2) und/oder dem biokompatiblen Polymer (3) wirkverbunden sind, bevorzugt durch chemische Bindung, besonders bevorzugt durch kovalente Bindung.

13. Detektionsvorrichtung (1) nach wenigstens einem der vorangegangenen Ansprüche, hergestellt durch:
a. Bereitstellung eines Trägers (2), und
b. chemische Aktivierung des Trägers (2) durch chemische, vorzugsweise kovalente Bindung der Detektionsrezeptoren (12) direkt oder über ein biokompatibles Polymer (3) an funktionelle Gruppen des Trägers (2), vorzugweise an organische funktionelle Gruppen des Trägers (2), bevorzugt über schwefel- und/oder stickstoffhaltige Verbindungen.

## Claims

1. Detection device (1) for in vivo and/or in vitro enrichment of sample material, comprising a biocompatible polymer (3) and a functional surface (10) equipped with detection receptors (12), the biocompatible polymer (3) forming the functional surface and the functional surface (10) comprising a three-dimensional structure having mutually facing functional portions (11) which form at least one intermediate space (13) that can be penetrated by a sample liquid, the detection device (1) comprising a carrier (2), **characterised in that** the carrier (2) comprises a coating (22) of metal that is applied to the carrier (2) by means of a galvanic process, and **in that** the biocompatible polymer (3) comprises saturated atom groups and covalently bonded detection receptors (12).

2. Detection device (1) according to claim 1, **characterised in that** the functional surface (10) is three-dimensionally structured in the macroscopic and/or in the microscopic range.

3. Detection device (1) according to at least one of the preceding claims, **characterised in that** the intermediate space (13) is channel-shaped at least in portions, a plurality of intermediate spaces preferably forming a branched network of channels.

4. Detection device (1) according to at least one of the preceding claims, **characterised in that** the functional surface (10) is formed having elevations, depressions and/or branched portions, and/or has a spiral-shaped, twist-shaped, coil-shaped, undulating, helical, filamentous, brush-shaped, comb-shaped, reticular, porous or sponge-like structure at least in part.

5. Detection device (1) according to at least one of the preceding claims, **characterised in that** the detection receptors (12) comprise antibodies, antibody fragments, amino acid structures, nucleic acid structures and/or synthetic structures having specific affinity to cell surfaces, preferably monoclonal antibodies of murine origin, chimeric antibodies or humanised antibodies, preferably HLA-G and/or EpCAM antibodies.

6. Detection device (1) according to at least one of the preceding claims, **characterised in that** the detection device (1) is formed at least in portions as a guide wire, stent and/or catheter.

7. Detection device (1) according to at least one of the preceding claims, **characterised in that** the detection device (1) comprises a functionalised portion (1 a) provided with the functional surface (10) and a non-functionalised portion (1 b) which are both joined to form a stylet.

8. Detection device (1) according to claim 7, **characterised in that** the non-functionalised portion (1 b) comprises a marking (1 c) for checking application and/or a rounded end (1 d) for protection from injury.

9. Detection device (1) according to at least one of the preceding claims, **characterised in that** the carrier (2) meets at least one of the following requirements:
a. the surface (20) of the carrier (2) is formed with elevations, depressions and/or branched portions and/or has a spiral-shaped, twist-shaped, coil-shaped, undulating, helical, filamentous, brush-shaped, comb-shaped, reticular, porous, sponge-like or similar structure at least in part,
b. the carrier (2) comprises a substrate (21) which is formed having elevations, depressions and/or branched portions and/or has a spiral-shaped, twist-shaped, coil-shaped, undulating, helical, filamentous, brush-shaped, comb-shaped, reticular, porous, sponge-like or similar structure at least in part,
c. the carrier (2) comprises a coating (22) of a metal from the 10th or 11th group of the periodic table of elements, preferably of nickel, copper, palladium, silver, platinum and/or gold,
d. the carrier (2) comprises functional groups, preferably organic functional groups, preferably sulfur-containing and/or nitrogen-containing functional groups, particularly preferably a coating (23) having functional groups,
e. the carrier (2) comprises a biocompatible dye, preferably a coating of a biocompatible dye,
f. the carrier (2) is in the form of a solid body or a hollow body,
g. the carrier (2) is made of a biocompatible material,
h. the carrier (2) is made at least in part of metal, preferably high-grade steel, medical high-grade steel or titanium; of glass, preferably glass fibre; of plastics material, preferably a foamed plastics material, a polymer, preferably polyethylene, polypropylene, polyurethane, polytetrafluoroethylene, a plastics material based on organic polymers, or a combination of said materials,
i. the carrier (2) comprises saturated atom groups and covalently bonded ligands and receptors,
j. the carrier (2) comprises and/or forms the functional surface (10).

10. Detection device (1) according to at least one of the preceding claims, **characterised in that** the biocompatible polymer (3) meets at least one of the following requirements:
a. the biocompatible polymer (3) is formed as a coherent polymer layer,
b. the biocompatible polymer (3) has a three-dimensional, preferably filamentous and/or porous, structure,
c. the biocompatible polymer (3) comprises a three-dimensional, preferably filamentous, and/or porous, surface,
d. the biocompatible polymer (3) has a carbon-containing, branched molecular structure,
e. the biocompatible polymer (3) is operatively connected to the carrier (2) preferably by means of functional groups, preferably by chemical bonding, particularly preferably by covalent bonding,
f. the biocompatible polymer (3) comprises functional groups, preferably carboxyl groups, the functional groups having an unbalanced molecule charge preferably as a result of chemical activation, the functional groups being preferably matched to the detection receptors (12),
g. the biocompatible polymer (3) has hydrophilic properties and is preferably a hydrogel,
h. the biocompatible polymer (3) comprises chemically and/or enzymatically cleavable groups,
i. the biocompatible polymer (3) is arranged in a cavity of the carrier (2),
j. the biocompatible polymer (3) is crosslinked,
k. the biocompatible polymer (3) comprises the functional surface (10).

11. Detection device (1) according to at least one of the preceding claims, **characterised in that** the functional surface (10) is coated with a protective layer (4), the protective layer (4) preferably meeting at least one of the following requirements:
a. the protective layer (4) is soluble in liquids, in particular in bodily fluids, preferably in blood,
b. the protective layer (4) is biocompatible,
c. the protective layer (4) is organically crystalline and comprises at least one of the following ingredients: alginates, preferably high-purity alginates, polyethylene glycols, cyclic and non-cyclic oligosaccharides, polysaccharides, antioxidative amino acids, proteins or vitamins.

12. Detection device (1) according to at least one of the preceding claims, **characterised in that** the detection receptors (12) are operatively connected to the functional surface (10), preferably to the carrier (2) and/or the biocompatible polymer (3), preferably by means of linkers or organic functional groups, preferably by chemical bonding, particularly preferably by covalent bonding.

13. Detection device (1) according to at least one of the preceding claims, produced by:
a. providing a carrier (2); and
b. chemically activating the carrier (2) by chemical, preferably covalent, bonding of the detection receptors (12) directly or by means of a biocompatible polymer (3) to functional groups of the carrier (2), preferably to organic functional groups of the carrier (2), preferably by means of sulfur-containing and/or nitrogen-containing compounds.

## Revendications

1. Dispositif de détection (1) pour assurer l'enrichissement in vivo et/ou in vitro de matériau d'échantillon, comprenant un polymère biocompatible (3) et une surface fonctionnelle (10) dotée de récepteurs de détection (12), le polymère biocompatible (3) formant la surface fonctionnelle et le surface fonctionnelle (10) présentant une structure tridimensionnelle avec des secteurs fonctionnels (11) dirigés les uns vers les autres, qui forment au moins un espace intermédiaire (13) pouvant être traversé par un liquide d'échantillon, et le dispositif de détection (1) comportant un élément de support (2), **caractérisé en ce que** l'élément de support (2) présente un revêtement (22) en métal qui est appliqué sur l'élément de support (2) par un procédé de dépôt électrolytique, et **en ce que** le polymère biocompatible (3) présente des groupes d'atomes saturés et des récepteurs de détection (12) liés de manière covalente.

2. Dispositif de détection (1) selon la revendication 1, **caractérisé en ce que** la surface fonctionnelle (10) est structurée de manière tridimensionnelle dans le domaine macroscopique et/ou microscopique.

3. Dispositif de détection (1) selon l'une au moins des revendications précédentes, **caractérisé en ce que** l'espace intermédiaire (13) est d'une configuration en forme de canal au moins par secteurs, plusieurs espaces intermédiaires formant de préférence un réseau de canaux ramifié.

4. Dispositif de détection (1) selon l'une au moins des revendications précédentes, **caractérisé en ce que** la surface fonctionnelle (10) est réalisée avec des élévations, des creux et/ou des ramifications, et/ou comprend au moins en partie une structure en forme de spirale, en forme de vis, en forme de vis sans fin, de forme ondulée, en forme d'hélice, de forme filamenteuse, en forme de brosse, en forme de peigne, en forme de filet, poreuse, ou sous forme de mousse.

5. Dispositif de détection (1) selon l'une au moins des revendications précédentes, **caractérisé en ce que** les récepteurs de détection (12) comprennent des anticorps, des fragments d'anticorps, des structures d'acides aminés, des structures d'acide nucléique et/ou des structures synthétiques avec une affinité spécifique pour des surfaces de cellules, de préférence des anticorps monoclonaux d'origine murine, des anticorps chimérique ou des anticorps humanisés, en particulier des anticorps HLA-G et/ou EpCAM.

6. Dispositif de détection (1) selon l'une au moins des revendications précédentes, **caractérisé en ce que** le dispositif de détection (1) est réalisé au moins en partie en tant que fil-guide, stent et/ou cathéter.

7. Dispositif de détection (1) selon l'une au moins des revendications précédentes, **caractérisé en ce que** le dispositif de détection (1) comprend un tronçon fonctionnalisé (la) muni de la surface fonctionnelle (10), ainsi qu'un tronçon non fonctionnalisé (1b), qui sont regroupés et assemblés en un stylet.

8. Dispositif de détection (1) selon la revendication 7, **caractérisé en ce que** le tronçon non fonctionnalisé (1b) présente une marque (1c) pour le contrôle d'application et/ou une extrémité arrondie (1d) en guise de protection à l'encontre de blessures.

9. Dispositif de détection (1) selon l'une au moins des revendications précédentes, **caractérisé en ce que** l'élément de support (2) satisfait à au moins l'une des exigences suivantes :
a. La surface (20) de l'élément de support (2) est réalisé avec des élévations, des creux et/ou des ramifications, et/ou comprend au moins en partie une structure en forme de spirale, en forme de vis, en forme de vis sans fin, de forme ondulée, en forme d'hélice, de forme filamenteuse, en forme de brosse, en forme de peigne, en forme de filet, poreuse, sous forme de mousse ou une structure similaire.
b. L'élément de support (2) comprend un substrat (21) qui est réalisé avec des élévations, des creux et/ou des ramifications, et/ou comprend au moins en partie une structure en forme de spirale, en forme de vis, en forme de vis sans fin, de forme ondulée, en forme d'hélice, de forme filamenteuse, en forme de brosse, en forme de peigne, en forme de filet, poreuse, sous forme de mousse ou une structure similaire.
c. L'élément de support (2) comporte un revêtement (22) en un métal du 10° ou du 11° groupe du système périodique des éléments, de préférence en nickel, cuivre, palladium, argent, platine et/ou or.
d. L'élément de support (2) comprend des groupes fonctionnels, en particulier des groupes fonctionnels organiques, de préférence des groupes fonctionnels soufrés et/ou azotés, de manière particulièrement préférée un revêtement (23) avec des groupes fonctionnels.
e. L'élément de support (2) comporte un colorant biocompatible, de préférence un revêtement en un colorant biocompatible.
f. L'élément de support (2) est réalisé sous forme de corps massif ou de corps creux.
g. L'élément de support (2) est réalisé en un matériau biocompatible.
h. L'élément de support (2) est réalisé au moins en partie, en métal, de préférence un acier surfin inoxydable, de l'acier surfin inoxydable médical ou du titane ; en verre, de préférence en fibres de verre ; en matière plastique, de préférence une matière plastique sous forme de mousse, un polymère, de préférence du polyéthylène, polypropylène, polyuréthanne, polytétrafluoroéthylène, en une matière plastique à base de polymères organiques, ou en une combinaison de ces matériaux.
i. L'élément de support (2) comprend des groupes d'atomes saturés et des ligands et récepteurs liés de manière covalente.
j. L'élément de support (2) comporte et/ou forme la surface fonctionnelle (10).

10. Dispositif de détection (1) selon l'une au moins des revendications précédentes, **caractérisé en ce que** le polymère biocompatible (3) satisfait à au moins l'une des exigences suivantes :
a. Le polymère biocompatible (3) est réalisé sous la forme d'une couche de polymère continue.,
b. Le polymère biocompatible (3) présente une structure tridimensionnelle, de préférence filamenteuse et/ou poreuse.
c. Le polymère biocompatible (3) présente une surface tridimensionnelle, de préférence filamenteuse et/ou poreuse.
d. Le polymère biocompatible (3) présente une structure moléculaire ramifiée, carbonée.
e. Le polymère biocompatible (3) est lié de manière active à l'élément de support (2), de préférence par l'intermédiaire de groupes fonctionnels, de préférence par une liaison chimique, et de manière particulièrement préférée par une liaison covalente.
f. Le polymère biocompatible (3) comprend des groupes fonctionnels, de préférence des groupes carboxyles, les groupes fonctionnels présentant, de préférence par activation chimique, une charge moléculaire non équilibrée, et les groupes fonctionnels étant de préférence adaptés aux récepteurs de détection (12).
g. Le polymère biocompatible (3) présente des propriétés hydrophiles et est de préférence un hydrogel.
h. Le polymère biocompatible (3) comporte des groupes pouvant être scindés par voie chimique et/ou enzymatique.
i. Le polymère biocompatible (3) est agencé dans une cavité ou un espace creux de l'élément de support (2).
j. Le polymère biocompatible (3) est réticulé.
k. Le polymère biocompatible (3) comporte la surface fonctionnelle (10).

11. Dispositif de détection (1) selon l'une au moins des revendications précédentes, **caractérisé en ce que** la surface fonctionnelle (10) est revêtue d'une couche de protection (4), la couche de protection (4) satisfaisant de préférence à au moins l'une des exigences suivantes :
a. La couche de protection (4) est soluble dans des liquides, notamment des liquides corporels, de préférence dans le sang.
b. La couche de protection (4) est biocompatible.
c. La couche de protection (4) est cristalline organique et comprend au moins l'un des constituants suivants : des alginates, de préférence des alginates hautement épurés, des polyéthylène-glycols, des oligosaccharides cycliques et non cycliques, des polysaccharides, des acides aminés antioxydants, des protéines ou vitamines.

12. Dispositif de détection (1) selon l'une au moins des revendications précédentes, **caractérisé en ce que** les récepteurs de détection (12) sont liés de manière active, de préférence par l'intermédiaire de groupes de liaison ou liens ou de groupes fonctionnels organiques, à la surface fonctionnelle (10), de préférence à l'élément de support (2) et/ou au polymère biocompatible (3), de préférence par une liaison chimique, et de manière particulièrement préférée par une liaison covalente.

13. Dispositif de détection (1) selon l'une au moins des revendications précédentes, fabriqué par :
a. la préparation et la fourniture d'un élément de support (2), et
b. l'activation chimique de l'élément de support (2) en assurant la liaison chimique, de préférence covalente, des récepteurs de détection (12), directement ou par l'intermédiaire d'un polymère biocompatible (3), à des groupes fonctionnels de l'élément de support (2), de préférence à des groupes fonctionnels organiques de l'élément de support (2), de préférence par l'intermédiaire de composés soufrés et/ou azotés.
